# EUROPEAN PATENT SPECIFICATION

(11) **EP 1 543 735 B1**
(45) Date of publication and mention of the grant of the patent: **07.10.2015**
(21) Application number: 03029505.9
(22) Date of filing: 20.12.2003
(51) Int. Cl.: A61P 17/02

(54) **NUTRITIONAL COMPOSITION FOR WOUND HEALING**
LEBENSMITTELZUSAMMENSETZUNG FÜR DIE WUNDHEILUNG
COMPOSITION NUTRITIONNELLE DE CICATRISATION DES PLAIES

(43) Date of publication of application: 22.06.2005
(73) Proprietor: Nestec S.A., 1800 Vevey (CH)
(72) Inventor: Smola, Hans, Dr. Med., 1012 Lausanne (CH)
(74) Representative: Stephen, Paula-Marie

(56) References cited:
- EP-A- 0 764 405
- EP-A- 1 161 945
- WO-A-01/78532
- WO-A-03/013487
- WO-A-03/075903
- DE-A- 10 221 403
- US-A- 5 384 308

## Description

### Field of the invention

This invention relates to a nutritional composition for promoting wound healing, particularly the healing of chronic wounds such as pressure ulcers (decubitus).

### Background of the invention

In normal wound healing, there are three phases which overlap to some extent. Briefly, the first phase is inflammation in which the clot forms and stops the bleeding from blood vessels followed by extravasation of mononuclear blood cells which clean the wound and remove debris. The next phase is the granulation phase in which fibroblasts proliferate and accumulate in the wound and produce collagen to assist in wound closure. This phase is characterised by high metabolic activity. Finally, epithelial cells begin to cover the wound.

Delayed or impaired wound healing is a problem for health care professionals and patients as it results in increased treatment times and stays in healthcare facilities and distress to patients. The process of wound healing can be interrupted in any of the phases described above as a result of factors such as infection or malnutrition. The pressure ulcers which frequently afflict elderly and bed-ridden patients are a particular concern and these categories of patients are often found to be suffering from malnutrition. Indeed, all patients with acute or chronic wounds exhibit increased nutritional requirements, displaying a need for increased nutrients and energy as compared with individuals not challenged by such metabolic stresses. If these patients are malnourished before suffering wounds, the wounds may simply fail to heal.

In recent years, much attention has focused on the role of arginine in wound healing. This is discussed for example in USP 5,053,387 which discloses an enteral nutritional formulation in which 1 to 3% of the total energy intake is preferably provided by arginine. Similarly, EP 960 572 A discloses a nutritional composition suitable for the treatment and prevention of pressure ulcers which includes arginine as well as large amounts of vitamins C and E. The role of arginine is also discussed in USP 5,733,884 which discloses a method of providing nutrition to a patient with an acute or chronic wound using a composition in which at least 2% of the energy is provided by arginine and the same amount by proline. This patent hypothesises that arginine and proline have a synergistic effect in enhancing wound healing. Commercially, there are a number of products marketed as suitable for promoting wound healing on the basis that they contain high levels of arginine including CUBITAN® and ARGINAID®.

WO 03/013487 discloses the use of amino acid mixture containing high amount of proline (7-40 %) for wound healing in pharmaceuticals.

An adequate supply of arginine is clearly relevant to the wound healing process. However, arginine is also a precursor for the formation of nitric oxide which acts as a vasodilator and enhances growth hormone secretion. It is not desirable for critically ill individuals to be exposed to high amounts of nitric oxide and yet this will inevitably happen if such individuals receive nutritional supplements containing high levels of arginine - see, for example L. Cynober, Curr Opin Clin Nutr Metab Care. 6:189-93 2003. Moreover, it is quite likely that a high proportion of elderly, bedridden or critically ill patients at risk of developing pressure sores will also suffer from conditions for which high levels of nitric oxide are contra-indicated (J. Takala et al., N Engl J Med 341:785-792 1999).

### Summary of the Invention

In a first aspect, the present invention provides a nutritional composition for promoting wound healing comprising a protein source comprising free proline a lipid source and a carbohydrate source wherein no more than 1.8% of the total calories of the composition derive from arginine and wherein the protein source includes proline in an amount of at least 3% of the total calories of the composition and wherein the protein source constitutes at least 28 % of the total calories of the composition.

In a second aspect, the present invention provides a method of providing nutritional support to a patient with an acute or chronic wound comprising the step of administering a therapeutically effective amount of a nutritional composition comprising a protein source comprising free proline, a lipid source and a carbohydrate source wherein no more than 1.8% of the total calories of the composition derive from arginine and wherein the protein source includes proline in an amount of at least 3% of the total calories of the composition and wherein the protein source constitutes at least 28 % of the total calories of the composition.

In a third aspect, the present invention provides the use of a protein source comprising free proline, a lipid source and a carbohydrate source for the manufacture of a therapeutic formulation for promoting wound healing wherein no more than 1.8% of the total calories of the formulation derive from arginine and wherein the protein source includes proline in an amount of at least 3% of the total calories of the formulation and wherein the protein source constitutes at least 28 % of the total calories of the composition.

### Detailed Description of the Invention

Although the inflammatory phase of the wound healing process described above is critical, the present inventor believes that from a therapeutic/nutritional approach, attempts to modulate this phase carry high risks and that the granulation phase offers better potential for nutritional intervention. In this phase, new connective tissue is synthesised and more than 80% of this tissue is composed of collagen. Collagen is rich in the amino acids proline (about 22%) and glycine (about 33%) and the presence of these amino acids is rate limiting for collagen formation, that is to say, collagen cannot be efficiently formed if they are not available in sufficient quantity. However, the normal diet contains only about 3% in total of these amino acids and it will be appreciated that individuals who have suffered wounds may ingest even less of them, particularly as proline is not generally regarded as an essential dietary amino acid. In the case of individuals suffering from malnutrition for whatever reason, these shortages may be particularly pronounced The composition of the present invention is therefore supplemented with proline in a quantity sufficient to facilitate collagen synthesis. It is particularly suitable for the amelioration of pressure ulcers but may also be used in the management of acute wounds including before and after surgery.

The composition of the present invention does not need to be supplemented with arginine - of course some arginine is likely to be present as part of the protein source. However, it is widely believed that arginine also has a role in the inflammatory phase of wound healing and, for this reason, the composition of the present invention is preferably supplemented with small amounts of arginine subject always to the requirement that arginine must account for no more than 1.8% of the total calories of the composition..

The composition of the present invention contains sources of protein, lipids and carbohydrate and may be administered orally or enterally. The composition preferably provides about 1.25 kcal/ml.

Protein is essential to healing as tissue damage results in a catabolic response that includes a requirement for a larger proportion of total calories as protein than is required by the general population. Research suggests that enteral fortification employing large quantities of protein can accelerate the synthesis of visceral proteins and so the protein source of the present invention constitutes at least 28% of the total energy content of the composition.

A variety of different protein sources may be used including intact protein sources such as casein or whey as well as hydrolysed proteins, free amino acids and even mixtures of intact and hydrolysed proteins and/or free amino acids, in each case supplemented with free proline and, optionally, free arginine. Preferably, the protein source of the present invention is selected to yield the highest amount of proline in the proteins so as to minimise the amount that needs to be added as the free amino acid.

Preferably, proline constitutes at least 3.5% of the calories of the composition of the present invention. At this level of contribution to total calories, the composition will need to be supplemented by about 3.0% (by weight of the protein source) proline.

The total calories/gram of nitrogen of the composition of the invention is preferably about 160:1. The total non-protein calories/ gram of nitrogen is preferably about 110:1.

The composition of the present invention also includes a lipid source. Lipids or fats are the primary source of stored energy in the body and energy from fat metabolism is used in all normal cell functions. As far as wound healing is concerned, fat metabolism results in the formation of prostaglandins and other regulators of the inflammatory process. The lipid source used in the present invention preferably constitutes about 20% of the total energy content of the composition. Of this 20%, preferably about 8% is constituted by mono- and di-glycerides of fatty acids. The ratio of n-6 to n-3 fatty acids is preferably between 4:1 and 10:1, more preferably about 7:1.

The composition of the present invention also includes a carbohydrate source. Glucose is the primary fuel for cellular metabolism of many tissues including leucocytes, macrophages and fibroblasts all of which are involved in the wound healing process. Glucose is needed to meet the specific metabolic demands of wound healing. The carbohydrate source used in the present invention preferably constitutes about 50% of the total energy content of the composition. Suitable sources of carbohydrate are maltodextrin and sucrose. Preferably, the carbohydrate source is substantially free of lactose.

Vitamins, minerals and trace elements are also important in the wound healing process. Preferably, the composition of the present invention at least complies with the compositional criteria set out in Directive 1999/21/EC on Dietary Foods for Special Medical Purposes as regards these micronutrients. However, certain micronutrients are particularly important for wound healing and therefore the composition of the present invention preferably contains more than the recommended minimum levels of vitamins C and E, manganese, zinc and selenium.

A liquid, ready to use composition according to the present invention will now be given by way of example:-

### Example 1

| | | |
|---|---|---|
| Caloric density | 1.25g/ml | |
| Protein | 30% of kcal | |
| of which (by weight): | -sodium caseinate | 50% |
| | milk protein concentrate | 45% |
| | free L-proline | 3% |
| | free L-arginine | 2% |
| total L-proline | 12.4% of protein source | |
| total L-arginine | 5.0% of protein source | |
| Caloric contribution of total proline | | 3.7% |
| Caloric contribution of total arginine | | 1.5% |
| Lipids | 20% of kcal | |
| of which | rapeseed oil | 35% |
| | corn oil | 34% |
| | soya oil | 20% |
| | mono and di-glycerides of fatty acids | 8% |
| | milk fat | 3% |
| n-6:n-3 | 7.2:1 | |
| Carbohydrate | 50% of kcal | |
| of which | corn syrup | 52% |
| | sucrose | 43% |
| | starch | 3% |
| | lactose | 2% |
| Vitamin C | 125mg/100ml | |
| Vitamin E | 7.5mg α-tocopherol equivalents/100ml | |
| Manganese | 1.9mg/100ml | |
| Zinc | 3.7mg/100ml | |
| Selenium | 19µg/100ml | |
| Osmolarity | 470 mosm/Kg water | |
| Water | 80.3% | |
| Density | 1.087g/ml | |
| Total cal/g nitrogen | 160:1 | |
| Non-protein cal/g nitrogen | 110:1 | |

As will be appreciated from the foregoing description, the composition may also contain other micronutrients of the type conventionally found in enteral compositions in accordance with EC Directive 1999/21/EC as well as flavourings such as coffee or vanilla, emulsifiers, thickeners and stabilizers of the type conventionally found in enteral compositions.

The nutritional composition may be produced by conventional methods. For example, the protein source and the lipid source are dissolved in water, preferably water which has been subjected to reverse osmosis, to form a liquid mixture. Emulsifiers may be dissolved in the lipid source prior to blending if desired. Preferably, a food grade emulsifier from a vegetable source is used.

The temperature of the water is conveniently about 50°C to about 80°C to aid dispersal of the ingredients. Commercially available liquefiers may be used to form the liquid mixture. Preferably, pH of the liquid mixture is adjusted to about 6.3 to 7 with food grade hydroxides.

After preparation of the liquid mixture, the carbohydrate source is added together with other easily dissolvable ingredients including, for example, vitamins, minerals, flavourings and colorants.

The liquid mixture may then be thermally treated to reduce bacterial loads (pasteurized). This may be carried out by steam injection or by heat exchanger; for example a plate heat exchanger.

If a shelf-stable liquid composition is required, an ultra heat treatment (UHT) is preferably conducted after pre-heating to 50-85°C. For example, an indirect UHT treatment may be conducted at 140-155°C for 5-8s, in a tube heat exchanger. The liquid mixture may then be cooled to about 60°C to about 85°C; for example by flash cooling. The liquid mixture is then homogenized and the resulting homogenised milky liquid may be aseptically filled into suitable containers such as 200 ml cups for oral feeding. Aseptic filling of the containers may be carried out by cooling the liquid mixture.

If a powdered, reconstitutable formula is required, the homogenised mixture can be evaporated and dried to powder; for example by spray drying. Conventional procedures may be used.

### Experimental Example

Normal human fibroblasts were trypsinised and seeded in 12 well plates at a density of 10,000 cells/cm³. When confluent, the cells were transferred to a culture medium with an amino acid distribution and concentrations designed to mimic those in human serum as closely as possible. The cell cultures were divided into two categories, a control culture in which the culture medium contained 0.201 mM proline and an experimental sample in which the culture medium contained 0.592 mM proline. After 24 hours fibroblast-conditioned medium containing 100 microgram/ml beta-aminoproprionitrile to prevent cross-linking of collagen molecules in the cultures was collected. The conditioned medium was dotblotted to a nitrocellulose membrane and probed for collagen type I content with a polyclonal immune-absorbed antibody. The value shown for the proline-supplemented samples is relative to the controls set at 100%.

| **Sample** | **% of control value** |
|---|---|
| Control (0.201 mM Proline) | 100% |
| Proline-supplemented (0.502 mM Proline) | 150% ± 21.9% |

This experimental example shows that human fibroblasts respond to proline supplementation with a 50% increase in collagen synthesis. In this proline-supplemented medium, increased collagen synthesis is independent of the addition of growth factors or other mediators stimulating collagen transcription. It indicates an increased substrate requirement for efficient collagen synthesis.

## Claims

1. A nutritional composition for the use of promoting wound healing comprising a protein source comprising free proline, a lipid source and a carbohydrate source wherein no more than 1.8% of the total calories of the composition derive from arginine and wherein the protein source includes proline in an amount of at least 3% of the total calories of the composition, and wherein the protein source constitutes at least 28% of the total calories of the composition.

2. A nutritional composition for the use of promoting wound healing according to claim 1 wherein at least 3.5% of the total calories of the composition derive from proline.

3. A nutritional composition for the use of promoting wound healing according the claim 1 or 2 wherein 1.5% of the total calories of the composition derive from arginine..

4. A nutritional composition for the use of promoting wound healing according to any preceding claim which composition has an energy density of about 1.25kcal/ml.

5. The use of a protein source including free proline, a lipid source and a carbohydrate source in the manufacture of a nutritional composition for providing nutritional support to a patient with an acute or chronic wound wherein no more than 1.8% of the total calories of the composition derive from arginine and wherein the protein source includes proline in an amount of at least 3% of the total calories of the composition, and wherein the protein source constitutes at least 28% of the total calories of the composition.

6. The use of claim 5 wherein at least 3.5% of the total calories of the composition derive from proline.

7. The use of claim 5 or 6 wherein 1.5% of the total calories of the composition derive from arginine.

## Patentansprüche

1. Nahrungszusammensetzung zur Verwendung bei der Förderung der Wundheilung aufweisend eine Proteinquelle aufweisend freies Prolin, eine Lipidquelle und eine Kohlenhydratquelle, wobei nicht mehr als 1,8% der Gesamtkalorien der Zusammensetzung aus Arginin stammen und wobei die Proteinquelle Prolin in einer Menge von mindestens 3% der Gesamtkalorien der Zusammensetzung enthält, und wobei die Proteinquelle mindestens 28% der Gesamtkalorien der Zusammensetzung aufweist.

2. Nahrungszusammensetzung zur Verwendung bei der Förderung der Wundheilung nach Anspruch 1, wobei mindestens 3,5% der Gesamtkalorien der Zusammensetzung aus Prolin stammen.

3. Nahrungszusammensetzung zur Verwendung bei der Förderung der Wundheilung nach Anspruch 1 oder 2, wobei 1,5% der Gesamtkalorien der Zusammensetzung aus Arginin stammen.

4. Nahrungszusammensetzung zur Verwendung bei der Förderung der Wundheilung nach einem der vorhergehenden Ansprüche, wobei die Zusammensetzung eine Energiedichte von etwa 1,25 kcal/ml hat.

5. Verwendung einer Proteinquelle einschließlich freies Prolin, einer Lipidquelle und einer Kohlenhydratquelle bei der Herstellung einer Nahrungszusammensetzung zur Bereitstellung einer Ernährungsunterstützung an einen Patienten mit einer akuten oder chronischen Wunde, wobei nicht mehr als 1,8% der Gesamtkalorien der Zusammensetzung aus Arginin stammen und wobei die Proteinquelle Prolin in einer Menge von mindestens 3% der Gesamtkalorien der Zusammensetzung enthält, und wobei die Proteinquelle mindestens 28% der Gesamtkalorien der Zusammensetzung aufweist.

6. Verwendung nach Anspruch 5, wobei mindestens 3,5% der Gesamtkalorien der Zusammensetzung aus Prolin stammen.

7. Verwendung nach Anspruch 5 oder 6, wobei 1,5% der Gesamtkalorien der Zusammensetzung aus Arginin stammen.

## Revendications

1. Composition nutritionnelle destinée à une utilisation pour favoriser la cicatrisation de plaie comprenant une source de protéines comprenant de la proline libre, une source de lipides et une source de glucides, dans laquelle pas plus de 1,8% des calories totales de la composition dérivent d'arginine, et dans laquelle la source de protéines comprend de la proline en une quantité d'au moins 3% des calories totales de la composition, et dans laquelle la source de protéines constitue au moins 28% des calories totales de la composition.

2. Composition nutritionnelle destinée à une utilisation pour favoriser la cicatrisation de plaie selon la revendication 1, dans laquelle au moins 3,5% des calories totales de la composition dérivent de proline.

3. Composition nutritionnelle destinée à une utilisation pour favoriser la cicatrisation de plaie selon la revendication 1 ou 2, dans laquelle 1,5% des calories totales de la composition dérivent d'arginine.

4. Composition nutritionnelle destinée à une utilisation pour favoriser la cicatrisation de plaie selon l'une quelconque des revendications précédentes, laquelle composition présente une densité d'énergie d'environ 1,25kcal/ml.

5. Utilisation d'une source de protéines comprenant de la proline libre, d'une source de lipides et d'une source de glucides dans la fabrication d'une composition nutritionnelle destinée à fournir un support nutritionnel à un patient avec une plaie aigue ou chronique, dans laquelle pas plus de 1,8% des calories totales de la composition dérivent d'arginine, et dans laquelle la source de protéines comprend de la proline en une quantité d'au moins 3% des calories totales de la composition, et dans laquelle la source de protéines constitue au moins 28% des calories totales de la composition.

6. Utilisation selon la revendication 5, dans laquelle au moins 3,5% des calories totales de la composition dérivent de proline.

7. Utilisation selon la revendication 5 ou 6, dans laquelle 1,5% des calories totales de la composition dérivent d'arginine.
